# EUROPEAN PATENT APPLICATION

(11) **EP 4 342 976 A1**
(43) Date of publication of application: **27.03.2024**
(21) Application number: 22943842.9
(22) Date of filing: 02.12.2022
(51) Int. Cl.: C12N 1/00, A01N 59/02, A01P 1/00, C12N 1/20

(54) **SOLUTION FOR DESTROYING BIOFILM AND METHOD FOR PRODUCING SAME**

(30) Priority: 24.05.2022 JP 2022084640
(71) Applicant: Oct, Inc., Kobe-shi Hyogo, 650-0047 (JP); Misera Co., Ltd., Yokohama-shi Kanagawa, 225-0002 (JP)
(72) Inventor: TANAKA, Yoshiro, KOBE-SHI Hyogo 650-0047 (JP); MIURA, Naoyoshi, YOKOHAMA-SHI Kanagawa 225-0002 (JP)
(74) Representative: VKK Patentanwälte PartG mbB
(86) International application number: PCT/JP2022/044506
(87) International publication number: WO 2023/228443

(57) **Abstract**

**[Problem to be solved]**

To provide a solution which is gentle on the human body and which effectively destroys biofilms produced by bacteria and other microorganisms attached to a substrate, and a method of producing thereof.

**[Solution]**

Provided is a solution for destroying biofilms, comprising water (H₂O) and deuterated sulfuric acid (D₂SO₄), with a pH of 0.5 or more and 4.0 or less. The solution for destroying biofilms can effectively destroy biofilms due to its good permeability. In addition, the solution for destroying biofilms can be obtained by diluting deuterated sulfuric acid (D₂SO₄) with water (H₂O) and adjusting the pH to 0.5 or more and 4.0 or less.

## Description

### [Technical Field]

The present invention relates to a solution for destroying biofilms and a method of producing thereof.

### [Background Art]

A biofilm is a membrane surrounding bacteria that forms when bacteria and other microorganisms attached to a substrate secrete extracellular polysaccharide (EPS, extracellar polysaccharide). EPS acts as a barrier to protect bacteria, as a transport pathway for bacteria to take in nutrients and the like, and to protect bacteria and other microorganisms within the biofilm from environmental changes and chemical substances.

Within a biofilm, a matrix of its higher-order structure, microcolonies of confined bacteria and other microorganisms are scattered about. Bacteria and a wide variety of microorganisms, such as fungi, algae and protozoa could be present in the microcolonies. When the number of bacteria and other microorganisms in a biofilm exceeds a certain level, the bacteria and other microorganisms in the biofilm are released from the biofilm, resulting in the spread of biofilms to other sites, thus spreading of contamination and infection.

Biofilms can be found in natural fields such as ponds and rivers, and in environments such as buildings and drainage pipes. And a hospital environment is no exception. Biofilms form not only in the human body but also in the environment. It has become a major medical problem, especially in the medical field where biofilms are involved in chronic, intractable infections.

The formation of biofilms by bacteria and other microorganisms can significantly reduce the effectiveness of medical agents, and bacteria and other organisms can themselves develop resistance to medical agents. The mechanism of resistance development to medical agents is thought to be due to the formation of a biofilm, which reduces the ability of antimicrobial agents to physically penetrate the biofilm, binding the antimicrobial agents to the matrix and reducing the efficacy of the medical agents. It is also thought that the deep layers within the biofilm become nutrient-poor and hypoxic, causing the bacteria and other microorganisms themselves to go dormant.

For example, antibiotic resistance increases hundreds of times more in biofilm-formed Pseudomonas aeruginosa than in floating cells, making medical treatment more difficult. In dentistry, bacteria produce biofilms in periodontal pockets, causing a difficult-to-treat periodontal disease.

The development of medical agents to inhibit biofilm formation has progressed. Treatment of infectious and other diseases becomes difficult once biofilm has formed. Efforts have therefore been made to develop medical agents that destroy formed biofilms. The invention described in Patent Literature 1 is intended to destroy biofilms produced by Cutibacterium acnes and contains at least one component for destroying biofilms produced by Cutibacterium acnes selected from the group consisting of isopropyl methylphenol, salicylic acid, glycyrrhizic acid, ibuprofen piconol, sulfadiazine and salts thereof, ethanol, resorcinol, sulfur and benzoyl peroxide.

The invention described in Patent Literature 2 relates to a method of destroying biofilms in patients with cystic fibrosis and uses isolated or recombinant integration host factor (IHF) polypeptides or respective fragments or equivalents thereof.

These medical agents for destroying biofilms were invented in consideration of the properties of biofilms produced by specific bacteria and other microorganisms. The medical agent for destroying biofilms described in Patent Literature 3 is intended to suppress halitosis and contains sodium hydrogen carbonate and hypochlorous acid as essential components. The medical agent is characterized by the use of an inorganic material with a relatively small molecular weight (molecular weight of 200 or less) so that it can pass through the water channels that exist on the surface layer of biofilms and through which blood components, the source of nutrients for bacteria and other microorganisms, pass. It is also described that at the center of the biofilms, where bacteria and other microorganisms exist in large numbers, there are even smaller holes called nanochannels through which only disinfectants and the like with ultra-low molecular weights of 100 or less can pass.

### [Prior Art Documents]

### [Patent Documents]

[Patent Literature 1] Japanese Laid-Open Patent Publication No. 2021-165284
[Patent Literature 2] Japanese Laid-Open Patent Publication No. 2020-037595
[Patent Literature 3] International Publication No. WO 2010-004699

### [Summary of the Invention]

### [Problems to be Solved by the Invention]

A biofilm is composed of a wide variety of components, including polysaccharides, proteins, nucleic acids and lipids, and the amount and biochemical properties of each biofilm-producing component vary among different bacterial species and strains. Since the medical agents of Patent Literature 1 and 2 above are characterized in that they destroy biofilms according to the properties of individual bacteria and other microorganisms and the properties of biofilms, there is a problem that they are not applicable to biofilms in general. The technical concept of Patent Literature 3 above is that the medical agent is delivered through water channels and nanochannels that biofilms generally have. However, according to the description in Patent Literature 3, the chlorine concentration of a solution containing hypochlorous acid as the main component must be several hundred ppm and must be washed away immediately after use.

The object of the present invention is to provide a solution which is gentle on the human body and which effectively destroys biofilms, and a method of producing thereof.

### [Means to Solve the Problem]

The present invention (1) is a solution for destroying biofilms, comprising water (H₂O) and deuterated sulfuric acid (D₂SO₄), with a pH of 0.5 or more and 4.0 or less.

The present inventors have studied the structural characteristics of biofilms and have succeeded in obtaining the solution of the present invention, that effectively destroys biofilms themselves and is also gentle on the human body. A biofilm is an extracellular polymeric substance (extracellular polysaccharide (EPS)) that contains polysaccharides and proteins, as well as macromolecules such as DNA, lipids and humic substances. In general, exopolysaccharides are composed of monosaccharides and non-carbohydrate substituents.

The solution of deuterated sulfuric acid (D₂SO₄) diluted with water (H₂O) and adjusted to a pH of 0.5 or more and 4.0 or less is a highly acidic solution, having a function to oxidize extracellular polysaccharides (EPS) and easily pass through water channels and nanochannels of a biofilm to decompose extracellular polysaccharides within the biofilm. By using deuterated sulfuric acid (D₂SO₄) as a component that increases the concentration of hydrogen ions to achieve a low pH, a solution can be made that is gentle on the human body and effectively destroys biofilms.

The upper limit of the pH of the diluted solution is preferably 4.0 or less, and more preferably 3.0 or less, 2.0 or less, 1.9 or less, 1.8 or less, 1.7 or less, 1.6 or less, 1.5 or less, 1.4 or less, 1.3 or less, 1.2 or less and 1.1 or less, in order of increasing preference. This is because the efficacy of the solution for destroying biofilms of the present application in degrading extracellular polysaccharides (EPS) increases with decreasing pH. The lower limit of pH is preferably 0.5 or more to be effective in degrading biofilms. In consideration of the effect on cells, the lower limit of pH is more preferably 0.6 or more, 0.7 or more, 0.8 or more, 0.9 or more and 1.0 or more, in order of increasing preference.

Polysaccharides, components of a biofilm, are a substance composed of a number of linked monosaccharides such as glucose, and can be degraded by a low pH solution through a mechanism called acid hydrolysis. In particular, polysaccharides are efficiently degraded at a pH of 2 or less. And the adverse effect (damage) to cellular tissue can be reduced by using deuterated sulfuric acid (D₂SO₄).

The present invention (2) is a method of producing a solution for destroying biofilms, wherein deuterated sulfuric acid (D₂SO₄) is diluted with water (H₂O) to comprise water (H₂O) and deuterated sulfuric acid (D₂SO₄), with a pH of 0.5 or more and 4.0 or less.

The method of producing a solution for destroying biofilms of the present invention (2) is a simple method of production because it is prepared only by diluting deuterated sulfuric acid (D₂SO₄) with water (H₂O) to give a pH of 0.5 or more and 4.0 or less.

### [Effect of the invention]

The present invention can provide a solution which is gentle on the human body and which effectively destroys biofilms, and a method of producing thereof.

### [Brief description of the drawings]

[Figure 1] Figure 1 shows the tendency of polysaccharides to degrade as the pH of the solution for destroying biofilms changes.
[Figure 2] Figure 2 shows the test results of adverse effects using hibiscus leaves.
[Figure 3] Figure 3 shows photographs of Experiments 1 to 3 using Streptococcus mutans.
[Figure 4] Figure 4 shows the results of Experiment 1 using Streptococcus mutans.
[Figure 5] Figure 5 shows the results of Experiment 2 using Streptococcus mutans.
[Figure 6] Figure 6 shows the results of Experiment 3 using Streptococcus mutans.
[Figure 7] Figure 7 shows the results of Experiment 4 using Fn bacteria.

### [Description of Embodiments]

The best embodiments of the present invention are described below with reference to the figures and tables. The sterilizing solution of the present invention is prepared by adjusting the pH of deuterated sulfuric acid (D₂SO₄) using water (H₂O). Deuterated sulfuric acid (D₂SO₄) manufactured by Cambridge Isotope Laboratories, Inc. was used. Tap water was used for water (H₂O). The solution for destroying biofilms of the present invention is composed of these two substances, and trace components which have no or little effect on the pH value may be added. The pH was measured using a model METRO-51 manufactured by HORIBA, Ltd.

### (Polysaccharide degradation experiment by pH change)

A preliminary test was conducted to examine how polysaccharides are degraded by the present invention, using starch syrup and vegetable jelly as polysaccharide alternatives to actual extracellular polysaccharides (EPS). The starch syrup used was manufactured by KATO SANGYO CO., LTD. (product name "Kanpy starch syrup") and the vegetable jelly used was from COOP (manufacturer: Ina Food Industry Co., Ltd.) (product name "Coop Agar").

Starch syrup is a mucoid sweetener produced by saccharifying starch with acid or diastatic enzymes and is a mixture of glucose, maltose and dextrin, with maltose being the main component. Coop Agar is a vegetable jelly mix with agar as the primary ingredient. The ingredients of Coop Agar are glucose, agar, konjac flour, thickeners, (locust bean gum, xanthan gum) and calcium lactate. The basic ingredient for both is polysaccharides.

Solutions for destroying biofilms adjusted to various pH values and a test solution were added to test mixtures of starch syrup and Coop Agar and the viscosity of each mixture was measured. Viscosity was measured using a "Viscotester (high viscosity type) VT-06" manufactured by RION Co., Ltd.

First, starch syrup and Coop Agar were mixed in a beaker at a ratio of 34 to 5 and the viscosity was measured with a Viscotester (high viscosity type) VT-06. The viscosity was 460 dPa s. The mixture of starch syrup and Coop Agar to be used for the test (test sample) was prepared weighing 150 grams. The pH values of the test solutions prepared by adjusting the pH of deuterated sulfuric acid (D₂SO₄) with water (H₂O) were 0.5, 2.0, 3.0, 4.0, 5.0 and 6.0. In addition, a test solution was prepared from tap water without the addition of deuterated sulfuric acid (D₂SO₄) at pH 7.0. To 150 g of the test sample, 4 cc of the test solution was added and the measurement was performed with a Viscotester (high viscosity type) VT-06. The results are shown in Table 1 below. The room temperature was 25°C and humidity was 63% when the test was conducted.

**[Table 1]**

| pH of test solution | Viscosity (dPa·s) |
|---|---|
| 7.0 | 320 |
| 6.0 | 310 |
| 5.0 | 240 |
| 4.0 | 210 |
| 3.0 | 180 |
| 2.0 | 150 |
| 0.5 | 130 |

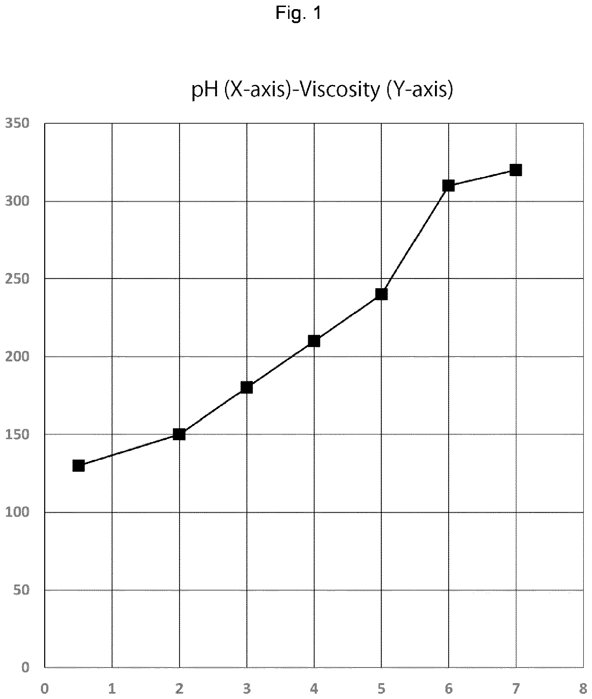

Figure 1 is a line graph of the measurement results from Table 1. The graph shows that as the pH decreases from pH 6, the viscosity also decreases. The test results clearly demonstrate that the polysaccharides are acid hydrolyzed by the low pH solution. At pH 0.5, more polysaccharides are degraded and the viscosity is reduced by almost 1/3 compared to pH 7.0.

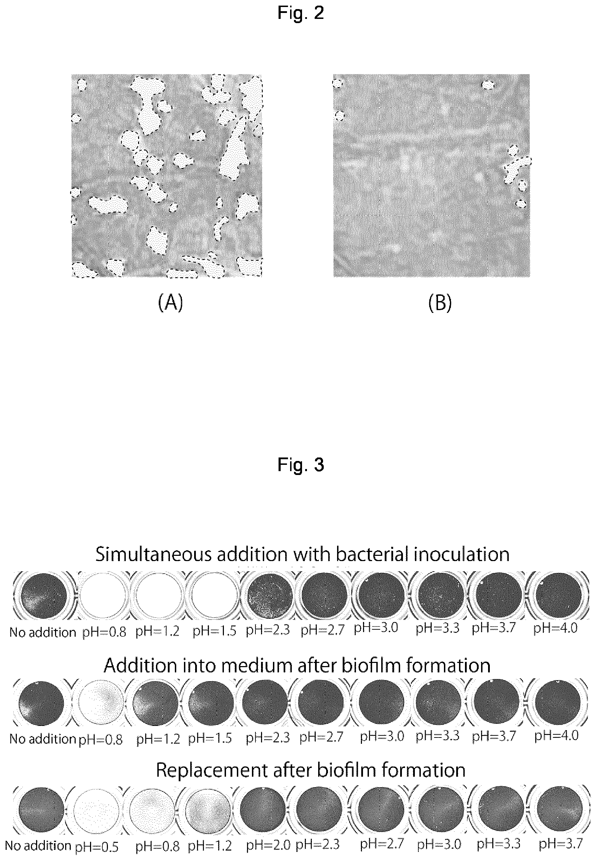

Figure 2 shows the experimental results of the safety of the solution for destroying biofilms of the present invention. A hibiscus leaf was divided into left and right halves by the central vein. The surface of one half was wetted with sulfuric acid (H₂SO₄) adjusted to pH 0.5 and the surface of the other half was wetted with deuterated sulfuric acid (D₂SO₄) adjusted to pH 0.5. The surfaces of the half leaves were observed after 20 hours. Figure 2(A) shows the leaf surface after 20 hours of wetting with sulfuric acid (H₂SO₄). The white spotted areas surrounded by dotted lines are areas where leaf cells are dead. Figure 2(B) shows the leaf surface after 20 hours of wetting with deuterated sulfuric acid (D₂SO₄). Compared to Figure 2(A), the white spotted areas surrounded by dotted lines are significantly smaller, indicating a high safety of the solution for destroying biofilms of the present invention.

Figures 3 to 6 show the experimental results in which Streptococcus mutans strains are actually used. Streptococcus mutans is a facultative anaerobe and a gram-positive streptococcus. It produces insoluble, sticky glucans that stick to tooth surfaces. It is one of the bacteria that mainly cause dental caries. THB (TODD-HEWITT BROTH) supplemented with 2% (w/v) sucrose was used as the medium. Sucrose is a sugar composed of the monosaccharides glucose and fructose with an α-1,2-glycosidic linkage and one of the disaccharides. THB manufactured by Becton, Dickinson and Company was used. For sucrose, "CASRN" manufactured by FUJIFILM Wako Pure Chemical Corporation was used. The plate used for culture was a Thermo Scientific Nunclon Delta Surface 96 well. The incubator used was a Panasonic multi-gas incubator MCO-5MUV-PJ. Culturing was conducted at 37°C under an atmosphere of 5% CO₂ gas, 5% O₂ gas and 90% N₂ gas.

Devices and the like used to measure residual biofilms are described below. For staining, 0.1% Crystal Violet from FUJIFILM Wako Pure Chemical Corporation was used. It was dissolved in distilled water to 0.1% (w/v).

Life Technologies EVOS XL Core was used as the microscope for photography. SH-1000 Lab from CORONA ELECTRIC Co., Ltd. was used as the absorption spectrometer. The data processing software was "SF6" and the absorbance wavelength was 590 nm.

### (Procedure for "Experiment 1: Simultaneous addition with bacterial inoculation")

Streptococcus mutans was diluted in 2% sucrose-containing THB and 10 inoculated samples of 100 µL each were plated onto cell culture plates (Thermo Scientific Nunclon Delta Surface 96well). At the same time as inoculation, 100 mL of test solutions of deuterated sulfuric acid (D₂SO₄) diluted with water (H₂O) and adjusted to pH 0.8, 1.2, 1.5, 2.3, 2.7, 3.0, 3.3, 3.7, and 4.0 were added. No test solution was added to one of the 10 inoculated samples.

The samples were then cultured in a multi-gas incubator for 24 hours to allow biofilms to form on the bottom of each plate. After culturing, the plates were washed with distilled water and stained with 0.1% Crystal Violet solution for 10 minutes. After staining, the plates were washed again with distilled water, dried and then observed and photographed with a bright field inverted microscope.

Crystal Violet was then extracted with 100 µL of 99.5% ethanol (FUJIFILM Wako Pure Chemical Corporation), and the absorbance was measured at a wavelength of 590 nm using an absorption spectrometer grating microplate reader.

### (Procedure of "Experiment 2: Addition into medium 24 hours after inoculation (after biofilm formation)")

Streptococcus mutans was diluted in 2% sucrose-containing THB and 10 inoculated samples of 100 µL each were plated onto cell culture plates (Thermo Scientific Nunclon Delta Surface 96well). The samples were then cultured in a multi-gas incubator for 24 hours to allow biofilms to form on the bottom of each plate.

Test solutions of deuterated sulfuric acid (D₂SO₄) diluted with water (H₂O) and adjusted to pH 0.8, 1.2, 1.5, 2.3, 2.7, 3.0, 3.3, 3.7 and 4.0 were added to the culture system 24 hours after the start of cultivation (after biofilm formation) without removing the medium. The samples were further cultured in a multi-gas incubator for 24 hours. After culturing, the plates were washed with distilled water and stained with 0.1% Crystal Violet solution for 10 minutes. After staining, the plates were washed again with distilled water, dried and then observed and photographed using a bright field inverted microscope.

Crystal Violet was then extracted with 100 µL of 99.5% ethanol (FUJIFILM Wako Pure Chemical Corporation), and the absorbance was measured at a wavelength of 590 nm using an absorption spectrometer grating microplate reader.

### (Procedure of "Experiment 3: Replacement of medium 24 hours after inoculation (after biofilm formation)")

Streptococcus mutans was diluted in 2% sucrose-containing THB and 10 inoculated samples of 100 µL each were plated onto cell culture plates (Thermo Scientific Nunclon Delta Surface 96well). The samples were then cultured in a multi-gas incubator for 24 hours to allow biofilms to form on the bottom of each plate.

The media were removed and the plates were washed with distilled water 24 hours after the start of cultivation. 100 mL of test solutions of deuterated sulfuric acid (D₂SO₄) diluted with water (H₂O) and adjusted to pH 0.5, 0.8, 1.2, 2.0, 2.3, 2.7, 3.0, 3.3 and 3.7 were added and the media removed were added back. The samples were further cultured in a multi-gas incubator for 24 hours. After culturing, the plates were washed with distilled water and stained with 0.1% Crystal Violet solution for 10 minutes. After staining, the plates were washed again with distilled water, dried and then observed and photographed using a bright field inverted microscope.

The upper panel of Figure 3 shows a photograph of Experiment 1, in which the test solutions were added at the same time as the Streptococcus mutans inoculation and the samples were cultured, followed by staining and washing. It indicates that the darker the color, the more biofilms remain. The color turns white at pH 0.8, 1.2 and 1.5, indicating that biofilm formation is significantly inhibited and destroyed.

The middle panel of Figure 3 shows a photograph of Experiment 2, in which the test solutions were added to the media 24 hours after the Streptococcus mutans inoculation (after biofilm formation) and the samples were cultured for another 24 hours, followed by staining and washing. The lower panel of Figure 3 shows a photograph of Experiment 3, in which the media were replaced with the test solutions 24 hours after the Streptococcus mutans inoculation (after biofilm formation) and the samples were cultured for another 24 hours, followed by staining and washing. These photographs show that the lower pH values are lighter in color, indicating that more biofilms were destroyed.

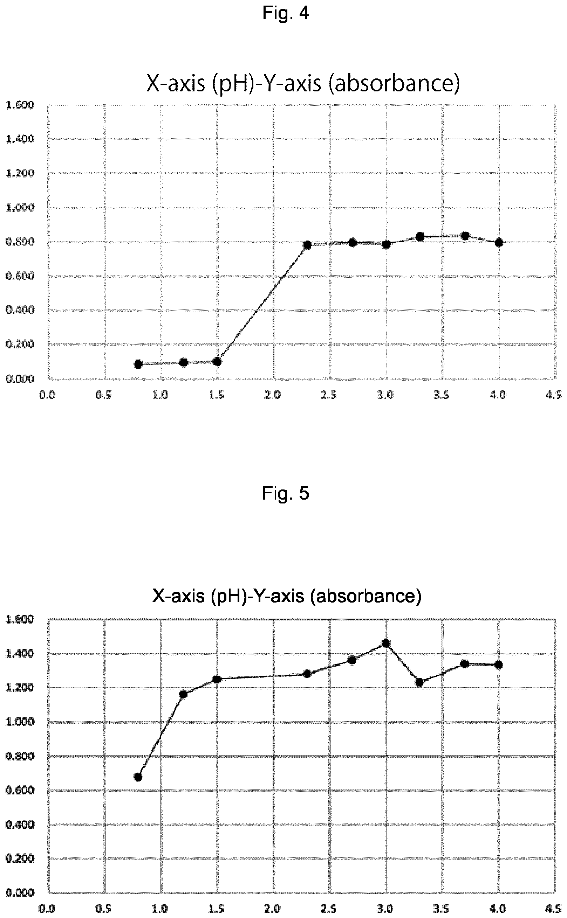

Figure 4 shows the results of the measured absorbance of the samples tested in Experiment 1 above. The horizontal axis is the pH value and the vertical axis is the absorbance. The absorbance decreased rapidly when the pH value was below 2.3, indicating that almost no biofilm was formed. Table 2 below shows the pH and absorbance data from Experiment 1.

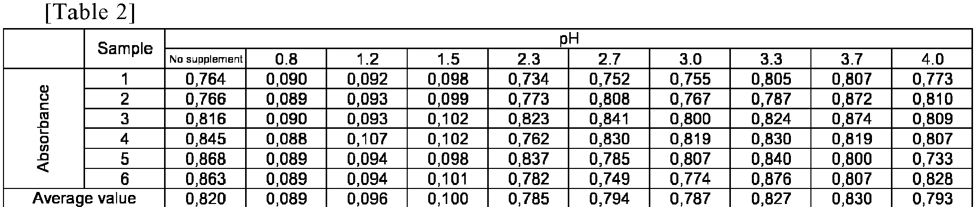

Figure 5 shows the results of the measured absorbance of the samples tested in Experiment 2 above. The horizontal axis is the pH value and the vertical axis is the absorbance. The absorbance decreased gradually when the pH value was below 3.0 and the absorbance decreased rapidly when the pH value was below 1.5. It indicates that more biofilms are destroyed when the pH is lower. Table 3 below shows the pH and absorbance data from Experiment 2.

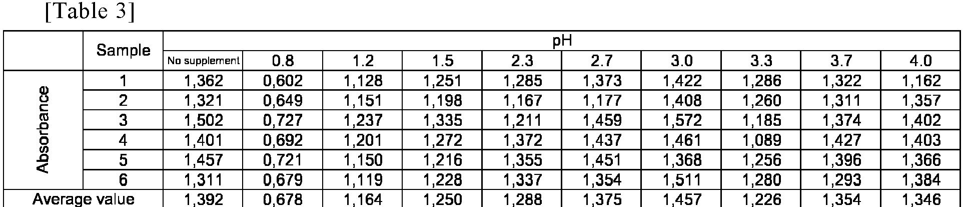

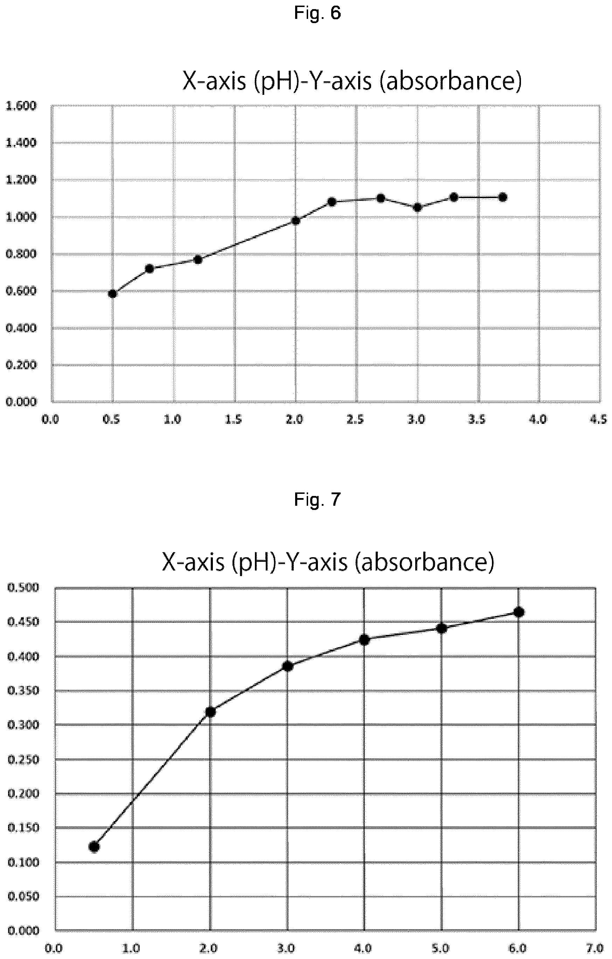

Figure 6 shows the results of the measured absorbance of the samples tested in Experiment 3 above. The horizontal axis is the pH value and the vertical axis is the absorbance. The absorbance decreased gradually when the pH value was below 2.3 and a marked decreasing trend continued until the pH reached 0.5. It clearly indicates that biofilms are destroyed when the pH is below 2.0. Table 4 below shows the pH and absorbance data from Experiment 3.

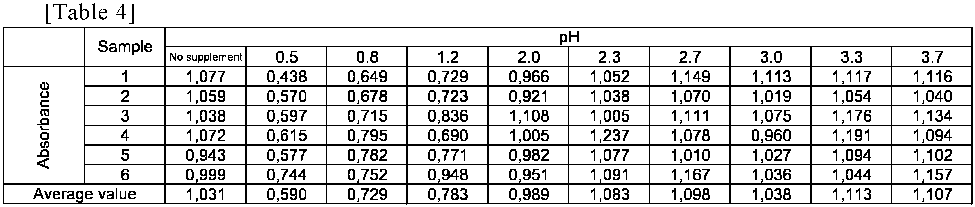

Since Streptococcus mutans produces acid using sugar as a nutrient source, the acid resistance properties are activated by the system to withstand a decrease in pH where the bacterial population density is high. Streptococcus mutans used in Experiments 1 to 3 is acid resistant. It is naturally effective against biofilms produced by biofilm-producing bacteria that do not have such a system.

Streptococcus mutans has the quorum sensing system and is resistant to particularly harsh environments. The quorum sensing system is a system which, when the number of bacteria exceeds a certain level, detects the bacterial population density and triggers signal transmission to optimize the environment for the bacteria.

Since Streptococcus mutans produces acid using sugar as a nutrient source, the acid resistance properties are activated by the system to withstand a decrease in pH where the bacterial population density is high. In addition, bacteriocin, a bactericidal substance, is produced to stop bacterial growth and regulate the number of bacteria.

In Streptococcus mutans, gene uptake is activated to adapt to the environment, so that it can easily mutate to be able to live in harsh environments.

Thus, Streptococcus mutans is difficult to kill even in a low pH environment due to the system, so a lower pH of 0.5 or more and 2.0 or less is required.

On the other hand, there are bacteria that, unlike Streptococcus mutans, are not acid resistant but still form biofilms. Examples include Fn bacterium (Fusobacterium nucleatum), which is a normal inhabitant in the oral cavity. This bacterium produces butyric acid, a cause of bad odor (halitosis), and is thought to cause periodontal disease. In recent years, the bacterium has also been found in the large intestine and is thought to cause colorectal cancer.

Unlike Streptococcus mutans, this bacterium does not have a quorum sensing system and is less acid-fast than Streptococcus mutans. The biofilms it produces can be destroyed even by a solution for destroying biofilms with a higher pH than that for Streptococcus mutans.

Figure 7 shows the experimental results in which Fn strains were actually used. THB (TODD-HEWITT BROTH) supplemented with 2% (w/v) sucrose was used as the medium. Sucrose is a sugar composed of the monosaccharides glucose and fructose with an α-1,2-glycosidic linkage and one of the disaccharides. THB manufactured by Becton, Dickinson and Company was used. For sucrose, "CASRN" manufactured by FUJIFILM Wako Pure Chemical Corporation was used. The plate used for culture was a Thermo Scientific Nunclon Delta Surface 96 well. The incubator used was a Panasonic multi-gas incubator MCO-5MUV-PJ. Culturing was conducted at 37°C under an atmosphere of 5% CO₂ gas, 5% O₂ gas and 90% N₂ gas.

### (Procedure for "Experiment 4: Simultaneous addition with bacterial inoculation")

Fn bacteria were diluted in 2% sucrose-containing THB and 10 inoculated samples of 100 µL each were plated onto cell culture plates (Thermo Scientific Nunclon Delta Surface 96well). At the same time as inoculation, 100 mL of test solutions of deuterated sulfuric acid (D₂SO₄) diluted with water (H₂O) and adjusted to pH 0.5, 2.0, 3.0, 4.0, 5.0 and 6.0 were added. No test solution was added to one of the 7 inoculated samples.

The samples were then cultured in a multi-gas incubator for 24 hours to allow biofilms to form on the bottom of each plate. After culturing, the plates were washed with distilled water and stained with 0.1% Crystal Violet solution for 10 minutes. After staining, the plates were washed with distilled water again and dried.

Crystal Violet was then extracted with 100 µL of 99.5% ethanol (FUJIFILM Wako Pure Chemical Corporation), and the absorbance was measured at a wavelength of 590 nm using an absorption spectrometer grating microplate reader.

Figure 7 shows the results of the measured absorbance of the samples tested in Experiment 4 above. The horizontal axis is the pH value and the vertical axis is the absorbance. When the pH value was below 4.5, the absorbance decreased compared to when the pH was 6.0, indicating that biofilm formation was made more difficult. Table 5 below shows the pH and absorbance data from Experiment 4.

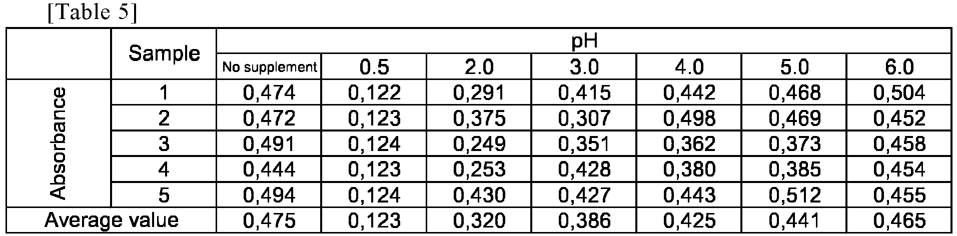

As shown in the results of Experiments 1 to 4, a solution for destroying biofilms with a pH adjusted between 0.5 and 2.0 is preferable for bacteria with strong acid fastness such as Streptococcus mutans, while even a solution with a pH of 4.0 or less is effective for bacteria with weaker acid fastness such as Fn bacterium. Therefore, the pH should be adjusted according to the type of bacterium of interest.

In Experiments 1 to 4, a solution for destroying biofilms composed of two components, water and deuterated sulfuric acid, was used. However, a solution containing other components such as surfactants, thickeners and colorants as sub-components can also destroy biofilms provided that the main components are water and deuterated sulfuric acid and the pH is between 0.5 and 4.

### [Industrial Applicability]

Since the solution for destroying biofilms of the present invention has the ability to powerfully destroy biofilms, the solution can be provided not only for biofilms occurring in the environment, but also for treatments in the dental and medical fields. It can also be provided to destroy biofilms formed on medical devices used in the medical field for more precise cleaning.

## Claims

1. A solution for destroying biofilms, comprising water (H₂O) and deuterated sulfuric acid (D₂SO₄), with a pH of 0.5 or more and 4.0 or less.

2. A method of producing a solution for destroying biofilms, wherein deuterated sulfuric acid (D₂SO₄) is diluted with water (H₂O) to comprise water (H₂O) and deuterated sulfuric acid (D₂SO₄), with a pH of 0.5 or more and 4.0 or less.

3. A method of destroying biofilms using the solution for destroying biofilms of Claim 1, by adjusting the pH according to the types of bacteria producing biofilms.
